# EUROPEAN PATENT APPLICATION

(11) **EP 2 677 030 A1**
(43) Date of publication of application: **25.12.2013**
(21) Application number: 12172851.3
(22) Date of filing: 21.06.2012
(51) Int. Cl.: C12N 11/08, C08G 18/76, C08J 9/00, C12R 1/645, C12N 9/00, C08G 18/36, C08G 18/38, C08G 18/66, C08G 18/64

(54) **Polyurethane rigid and flexible foams as composite obtained from wood origin raw materials and used as support for immobilization of microorganisms that produce ligninolytic enzymes**

(71) Applicant: Latvijas Valsts Koksnes kimijas instituts, Riga LV 1006 (LV); Carretera de Sacramento, s/n La Canada de San Urbano, University of Almeria, 04120 Almeria (ES)
(72) Inventor: Cabulis, Ugis, 1084 Riga (LV); Kirpluks, Mikelis, 3042 Brankas (LV); Stirna, Uldis, 1082 Riga (LV); Lazzeri, Andrea, 56126 Pisa (IT); Cinelli, Patrizia, 50144 Firenze (IT); Lopez Lopez, Maria Jose, 04007 Almeria (ES); Suarez Estrella, Francisca, 04003 Almeria (ES); Moreno Casco, Joaquin, 04720 Almeria (ES); Vargas Garcia, Maria del Carmen, 04008 Almeria (ES)
(74) Representative: Fortuna, Jevgenijs

(57) **Abstract**

The invention pertains to polyurethane rigid and flexible foams (PUR), which are obtainable from wood origin raw materials - tall oil derived polyols or lignin, said foams being used as support for immobilization of microorganisms, which produce ligninolytic enzymes.

## Description

### Field of the invention

The invention relates to polyurethane rigid and flexible foams obtained from wood origin raw materials; said foams being used as support for immobilization of microorganisms, which produce ligninolytic enzymes.

### Background of the invention

It is known that the polyurethane and polyisocyanurate foams widely are used as insulation material in refrigeration industries and as construction material in form of sandwich panels for finishing of prefabricated buildings, pipelines, freezers, refrigerators, as well as the said foam materials can be used to insulate buildings on the site. Polyurethane foam is produced by casting or spraying source material produced by mixing in the necessary ratio polyol component and polyisocyanate component. Polyol component consists of one or more polyols, blowing agent, foam stabilizer and a catalyst. Besides, as third component in polyurethane blowing process can be used natural fibres of different sizes and types. Natural fibres include: wood fibres, cellulose fibres, flax fibres, organic fibres and modified above mentioned fibres.

Mainly in the polyurethane foam production the polyether-type polyols or polyester-type polyols are used whose functionality is equal to or greater than 2. Polyols entering in the reaction with polyisocyanate component form the polyurethane foam polymer matrix.

In the polyurethane foam production variety of tertiary amines, potassium octoate or potassium acetate or tin containing organic compounds are used as catalysts. Commonly as foam stabilizers the polidimetilsiloxan blockpolymers are used designed to encourage mixing and stabilization of polyol component and isocyanate component in the cell blowing process. The foam can be got with an open or closed cell by varying the foam stabilizers. Even in the production of flexible polyurethane foams there are two main reactions: the blow reaction and the gelation reaction. If the gelation reaction (cross-linking) occurs too quickly it may result in close celled foam. If the blow reaction (gas-producing) occurs too quickly, the cells may open before the polymer has enough strength to maintain the cellular structure, resulting in collapse of the foam. These two reactions must be kept in proper balance in order to obtain the desired product.

In the preparation of polyurethane foams there is growing concern on the pollution due to the blowing agent required for their production. The blowing agent Chlorofluorocarbons (CFCs) had been the standard for years until banned by the Montreal Protocol because of their ozone depletion potential so researchers had to look for new alternative blowing agents. There are basically three types of blowing agents: water liquid chemicals with low boiling point, which evaporate due to the exothermic reaction of polyols and isocyanate, air which is blown into polyols and isocyanate mixture. Water entering in the reaction of an isocyanate component produces CO₂, which acts as a blowing agent. Hydro-fluor-carbons (HFC) or pentane type blowing agents rapidly evaporates at the influence of reaction heat that is released in the reaction of the isocyanate component and polyols or water, and/or in the result of isocyanate groups cyclotrimerization.

By varying of polyol components in the polyol mixture, there is wide opportunity to change the polyurethane foam physical and mechanical properties.

There is growing interest in the polyurethane foam production from renewable raw materials (Hill, 2000; Petrovic, 2008), among which an important place occupies vegetable oils (this is confirmed by patent of Petrovic, 2010 "Modified vegetable oils based polyols") or a variety of chemical by-products, for example pulp production by-product such as tall oil or lignin by products of wood industry and of bioethanol production. This interest in renewable raw materials is technologically and economically justified, as many of the vegetable oil polyols synthesis, and lignin in terms of price are competitive with polyols derived from petrochemical feedstock. Renewable raw materials for polyurethane foam production enable them to give a new set of properties. There are many inventions that are devoted to the production of polyurethane foam oleopolyols obtained from epoxidized vegetable oils, but most of oleopolyols are characterized by low hydroxyl values (from 150 to 250mgKOH/g) and for this reason these polyols have only greater use in flexible polyurethane foam production. As well lignin has been mainly addressed for the production of rigid polyurethane. The inventors have achieved synthetic processes which allow the production of rigid and flexible foams with the structure and composition particularly efficient for use as support for growth of microorganisms that produce ligninolytic enzymes. Most of the research on polyurethane produced by renewable resources mainly focuses on the use of soybeans oil via epoxidation followed by hydroxylation (Petrovic 2008). Polyols were reacted with diphenylmethane diisocyanate (DMI) to produce polyurethane in a paper titled "Structure-property relationships in different types of vegetable oils" (Guo, 2002); in "Rigid Polyurethane Foams Based on Soybean Oil" (Guo, 2000) by Guo et al, the soy polyols were prepared via oxirane ring-opening reaction of epoxidized soybean oil with methanol and blown with HCFC and pentane to produce rigid polyurethane foams. Several patents are present in the literature on the production of polyurethane from castor oil or similar vegetable oils such as "Biodegradable polyurethane foams prepared from renewable resources" (Kelly, 2001) by Kelly et al.; in this invention the low-index polyurethane foam, which is at least partially biodegradable, is made by a reaction product of 10-35% isocyanate; 90-65% active hydrogen-containing component selected from 80-100% component derived from a natural, renewable component (e.g., castor oil, corn syrup and sucrose) and 0-20% component derived from a petrochemical source e.g., glycerol, Arcol LG 650 (glycerol-propylene oxide polyether triol); 0.5-1% catalyst e.g., Dabco TMEDA (tetramethyl-1,2-ethylenediamine), Polycat 77 (bis(dimethylaminopropyl)methylamine) and Dabco TMR 2 (N-(2-hydroxypropyl)-N-trimethylammonium formate); from 0.3-3 surfactant (Tegostab B 8433, Ortegol 501, Tween 40 and Calsoft F 90) and 0.5-20% blowing agent (e.g., water). The rigid polyurethane foams absorb water rapidly and are useful as floral supports, growing media, etc. The similar patent "Preparation of polyurethane rigid foams" (Scherbel K, 1997), castor oil is used as polyol for preparation of rigid polyurethane foams for refrigerator industry, but due to low hydroxyl number of castor oil (165 ± 5 mgKOH/g) it is not used as main polyol in composition, the castor oil improve the solubility of pentane in described compositions.

In "Novel polyether polyols based on cashew nutshell liquid, a process for the production of these polyether polyols, flexible foams produced from these polyether polyols, and a process for the production of these foams" (Reese, 2008) by Reese et al. the invention relates to novel polyether polyols which are prepared by alkoxylation of renewable resource materials, and particularly cashew nutshell liquid (CNSL), and to a process for the preparation of these novel polyether polyols. This invention also relates to flexible polyurethane foams prepared from these long chain polyether polyols, and to a process for the production of these flexible polyurethane foams.

In "Hydrophobic polyurethane foams, their manufacture and use" (Jarre W., 1980) hydrophobic polyurethane foams which have densities of from 4 to 15 g/liter and in which the number of closed cells is from 2 to 30%, are obtained from polyisocyanates, polyhydroxy compounds, catalysts and water, with or without lipophilic compounds (obtained in oleic acid reaction with diethanolamines). The foams are suitable for the absorption of oil and/or by hydrophobic solvents, which may or may not contain halogen, from water.

In "Improved cellular plastic material" (Kurth, 2000) by Kurth, a cellular material consists of the reaction product of an A-side and a B-side, wherein the A-side is made by a diisocyanate and the B-side is made by a vegetable oil (e.g., soy oil), a crosslinking agent consist of a multi-functional alcohol and a catalyst.

The problem addressed by the invention of Mueller H.-P. (Mueller H.-P., 2000) is the provision of hydrophobic rigid polyurethanes and, in particular, rigid PUR foams which are distinguished by the absence of shrinkage, by dimensional stability, by hydrolytic stability, by excellent long-term behaviour and by very good insulation properties. Such rigid PUR foams should be obtained using water as the blowing agent, have closed cells and show the advantages mentioned above. Rigid polyurethanes are obtained by reacting (1) a polyisocyanate with (2) a polyol mixture made up of (a) an OH-functional polyester based on a C9-22 fatty acid and non-functional oligomeric polyolefin and optionally an at least difunctional polyol and/or polyamine. But in description of invention there are no data about long term storage stability of polyol system, it is possible that the non-functional oligomeric polyolefin in presence of water as foaming agent form emulsion.

In "Visco-elastic polyurethane foam with castor oil" (Klesczewski, 2009), a patent by Klesczewski et al., the invention relates to polyether polyol compositions containing renewable raw materials, and a process for preparing visco-elastic polyurethane foams using such compositions. Under the aspect of renewability, a complete or at least partial replacement of the synthetic polyether polyols by castor oil as a renewable raw material for the preparation of various polyurethane systems is achieved. The invention describes a process for the preparation of open-pore visco-elastic polyurethane flexible foams based on renewable raw materials by reacting: a) polyisocyanates with b) a polyol mixture consisting of bi) compounds having at least two isocyanate-reactive hydrogens and an OH number of 20 to 100 mg of KOH/g; and bii) compounds having at least two isocyanate-reactive hydrogens and an OH number of 100 to 800 mg of KOH/g; and biii) compounds having at least one and at most two isocyanate-reactive hydrogens and an OH number of 100 to 800 mg of KOH/g; and c) foaming agents; characterized in that each of components bi) and bii) contains at least one compound which contains renewable raw materials or their reaction products. Castor oil is more preferably employed as compound bii). A drawback of this process is the fact that the main component, i.e. bi), is a reaction product of a renewable raw material with epoxies, i.e., is also a polyether polyol ultimately; in particular, a chemically unaltered renewable raw material cannot be exclusively employed here.

Polyurethanes from renewable resources are also proposed for applications like carpets in "Polyurethane carpet backings made using hydroxymethylated polyester polyols" by Jenkines (Jenkines, 2006), the formulation allows a significant replacement of conventional polyols with polyols derived from annually renewable resources, while maintaining important properties like edge curl, tuft bind, viscosity and good curing rates. Such polyol can be obtained by reacting hydroxymethylated soybean oil with poly(ethylene oxide).

In "Flame retardant urea bio-based urethane composition" (Blount, 2006) by Blount, polyurethane compound is produced by mixing, selective heating, and reacting a component A consisting of urea and/or a urea condensate with a component B consisting of bio-based compounds (vegetable oils and any oil containing seeds), and C polyisocyanates. Moreover blowing agents, surfactants, urethane catalyst such as tertiary amines, fillers and any suitable carbonization auxiliaries are used in this invention. Carbonization auxiliaries are compound that in presence of fire assist the formation of carbonization foam or a char used in quantities of 0 to 300 percent by weight of the amino condensation compound.

In "Composition for preparing a biodegradable polyurethane-based foam and a biodegradable polyurethane foam" (Nascimento, 2009) by Nascimento et al., a composition for preparing a biodegradable polyurethane-based foam and a biodegradable polyurethane foam, comprising mixtures based on poly(hydroxybutyrate) polymer, polyols of renewable source, isocyanates and additives, is given. In the process, the poly(hydroxybutyrate) plus the polyol, the isocyanate and the additives are previously mixed in specific mixers; once the homogenization is reached, the mixture is poured in mould for growing. After curing, the resulting product presents foam characteristics, with its density, toughness and cell size varying according to the proportion of the reagents and allowing the manufacture of several products. These biodegradable polyurethane foam, comprises from about 4% to about 30% in mass of poly(hydroxybutyrate) or its poly-hydroxybutyrate-valerate copolymer; from about 15% to about 40% of a polyol of renewable source; from about 35% to about 55% of isocyanate; from about 1% to about 2% of at least one additive presenting one of the functions: catalyst, surfactant, pigmentation, filler and expansion.

Liquefied lignin was used by Hatakeyama et al., for the production of semi-rigid polyurethane foams in "Environmentally compatible hybrid type polyurethane foams containing saccharide and lignin components" (Hatakeyama, 2005), both lignin- and molasses-poly(ethylene glycol) polyols were mixed with various ratio and were reacted with MDI in the presence of silicone surfactant and catalyst, using water as foaming agent. More recently, lignin was investigated as potential source for producing polyurethanes. See for example, the paper titled "An integrated process to produce vanillin and lignin-based polyurethanes from Kraft lignin" (Borges, 2009) by Borges da Silva et al., where an integrated process that includes reaction and separation steps for producing vanillin and lignin-based polyurethanes from Kraft lignin is presented. After vanillin recovery the remaining lignin can act as a raw material to produce polyurethanes and/or biofuels.

In "Lignin-based polyurethane and process for producing the same" (Hatakeyama 2005), by Hatakeyama et al., describes a polyurethane that it is produced by polycondensation reaction of a polyol solution; the polyol solution contains, not only a glyceride, but also molasses or sugar and lignin material in dissolved form, with a polyisocyanate. The polyurethane of this patent is rigid foam which claims to be excellent in biodegradability and valuable mechanical properties.

In "Homogeneous stable polyhydroxyl compound containing lignin with good processing properties" (Hinz 2007) by Hinz et al., lignin-containing polyols are produced by reaction of alkylene oxide with mixtures containing lignin having Na and K ion content <5000 ppm, polyalkylene glycol having OH functionality ≥2, and, optionally, compounds having ≥1 of OH, NH, and/or NH2 functionality and reactive H functionality ≥2 and are used to prepare polyurethanes that exhibit improved elongation at break, tensile strength, and/or cut-growth resistance.

In "Lignin based polyols" (Kurple 1997) by Kurple, a rigid polyurethane product was prepared from 1.5 parts 300:700 lignin (prepared from a solvent pulping process) and WL 440 (polyether polyol of propylene oxide and ethylene oxide) and 1 part Rubinate RI 840 (isocyanate). In "Pressurized, blowing agent-containing isocyanate semi-prepolymer mixtures based on lignin-polyether polyols and their use for producing polyurethane foams" (Reese 1998) by Reese et al., the title compounds contain isocyanate prepolymers prepared from organic isocyanates (esp. crude MDI) and alkoxylated lignin with OH no. 30-80 (preferably in mixtures. with other polyols) and are useful for the preparation of one-component polyurethane foams with good dimensional stability, expansion shrinkage resistance.

In "New polyurethane and processing for preparing same" (Hirose 1989) by Hirose et al., isocyanates are used with cellulose, hemicellulose, and lignin in the preparation of polyurethanes. The polyurethane of this invention, containing a hard segment derived from at least one polyhydroxy compound selected from cellulose and lignocellulose, are claimed to be excellent in mechanical and thermal characteristics and very economical in cost with the possibility to tune properties varying the proportion of the hard segment and the soft segment derived from the polyol.

In "Method of producing prepolymers from hydroxyalkyl lignin derivatives" (Glasser 1992) and "Method of producing star-like polymers from lignin" (Glasser 1991) Glasser and co-authors reported a method of producing pre-polymeric materials from lignin (kraft, organosolv, steam exploded lignin) that has been hydroxaylkyl modified, so the lignin became substantially non phenolic and solvent soluble and/or liquid and then it is able to react with material which yield prepolymers to obtain a polymer. While in "Polyurethane intermediates and products and methods of producing same from lignin", (Glasser 1977) by Glasser et al., a polyol intermediate from lignin is produced by reacting lignin with maleic anhydride copolymers, which are then reacted with an oxyalkylating medium with or without prior hydrolysis to form the polyol intermediates. The polyester-ether intermediates react with an isocyanate medium having an isocyanate with at least 2 NCO groups per molecule to produce polyurethane products with or without simultaneous foaming.

Immobilization of ligninolytic microorganisms and enzymes. Immobilization refers to the action of the limiting movement or making of movement incapable. Biocatalysts can be immobilized either using the isolated enzymes or the whole cells. When whole cells of microorganisms are immobilized, the cells are kept in one place. Normally, in a bioreactor cells are floating around in nutrient liquid. In an immobilized cell bioreactor, the cells are trapped while nutrient flows over them Immobilization of whole cells has been shown to be a better alternative to immobilization of isolated enzymes (Mahmoud and Helmy, 2009). Compared with suspended microorganism, immobilized microorganism possesses many advantages, such as high biomass, high metabolic activity, improved availability of oxygen, efficient mass transfer and strong resistance to toxic chemicals and so on (Mahmoud and Helmy, 2009). Moreover, immobilization of biocatalysts helps in their economic reuse and in the development of continuous bioprocesses.

Ligninolytic enzymes are mainly lignin peroxidase (LiP), manganese peroxidase (MnP) and a copper containing phenoloxidase, known as laccase. These enzymes are produced by a wide range of microorganisms being the fungi, specially the white-rot fungi, the most efficient producers. These enzymes are involved in the degradation of the complex and recalcitrant polymer lignin and several phenolic and non-phenolic compounds, including many toxic organic pollutants, such as chlorophenols or polycyclic aromatic hydrocarbons. They are also capable of forming cross-links in biopolymers to produce materials by means of polymerization processes. Consequently, they, especially laccase, can display both degrading and polymerizing (cross-linking) abilities.

Large-scale production of ligninolytic enzymes has been difficult mainly owing to the sensitivity of the organism to agitation. However, as reported by Duran et al. in "Applications of laccases and tyrosinases (phenoloxidases) immobilized on different supports: A review", it has been shown that the immobilization of producing microorganism appears to stabilize the enzyme production system (Duran et al., 2002).

The selection of an adequate support for performing microorganism immobilization is essential, since the success of the process depends on it. In "Phanerochaete chrysosporium strains CNCM I-1511, I-1512 and I-1513 for producing lignin peroxidase and Manganese peroxidase" Mouka et al (Mouka et al., 1999) suggest several hydrophobic, hydrophilic or neutral supports that can be used for this purpose, the surface of which is preferably rough, and containing, for example, cavities, meshing (gauze or web), hollows or holes. These supports are arranged in ordered fashion or otherwise, fixed or mobile in the liquid phase consisting of the appropriate nutrient medium, and can have a variety of shapes (cylindrical or cubic, in one or several pieces). These supports, like the medium, may be replaced and/or renewed continuously or discontinuously during culture. As examples of supports, stoneware, glass, metal or plastic, polyurethane, polyester, polyacrylate, nylon and polyamide can be used. In the invention by Moukha et al. 1999 the fungus *P. chrysosporium* is immobilized in concentric cylinders made of metal gauze having a 0.55mm mesh. The strains used were hypersecretory of lignin peroxidase and manganese peroxidase.

The advantage of PUR foams for microorganisms immobilization relies on the easy control of the pore size, stable maintenance of the quantity of cells, and large-scale application at low price. As stated by Rodriguez Couto and Sanromán in "Application of solid-state fermentation to ligninolytic enzyme production" the success of immobilization greatly depends on the composition and structure of the PUR foam (Rodriguez Couto and Sanromán, 2005).

Immobilization of ligninolytic microorganisms is a subject of great interest and several reports are available in the scientific literature. In "Overproduction of lignin peroxidase by Phanerochaete chrysosporium (BKM-F-1767) under nonlimiting nutrient conditions" Dosoretz et al. (Dosoretz et al., 1993) reported a procedure for ligninolytic enzymes production by *Phanerochaete chrysosporium* BKM-F-1767 immobilized on polyurethane foam. The fungus was grown in a non-immersed liquid culture system under conditions close to those occurring in nature. Fungal mycelium was immobilized on porous pieces of polyurethane foam saturated with liquid medium and highly exposed to gaseous oxygen. The immobilization system consisted of cubes of polyurethane foam, approximately 0.5 cm per side, that were arranged such that the level of the liquid was about one-fourth of the total height of the bed. By using these conditions authors applied for a patent in "Methods for producing lignin peroxidase and its use in skin and hair lightening" (Belinky et al. 2009) in which *P. chrysosporium* ATCC 24725 is immobilized on polyurethane foam and the enzyme produced is proposed as component of cosmetic suitable for skin and air lightening. Similarly, Kwon et al. in "Optimized production of lignolytic manganese peroxidase in immobilized cultures of Phanerochaete chrysosporium" (Kown et al., 2008) propose immobilization of *P. chrysosporium* on cubic polyurethane foam as a means to increase ligninolytic enzymes production

In "Microbial method for producing lignin peroxidase" Asther et al. (1992) propose a method for producing lignin peroxidase from P. chrysosporium by cells immobilized on polyurethane in a medium comprising the addition of phospholipids, emulsified fatty acids and veratryl alcohol at different steps of culture and by variying the content of medium constituents.

In "The implication of Dichomitus squalens laccase isoenzymes in dye decolorization by immobilized fungal cultures" Susla et al. (Susla et al., 2007) immobilized *D. squalens* on polyurethane foam or pine wood in a bed reactor at laboratory scale. Authors found out that immobilization of fungal cultures on pine wood improved eminently laccase production in comparison to the liquid cultures and led to an effective decolorization of an anthraquinone dye Remazol Brilliant Blue R and an azo dye Reactive Orange 16. Fujita et al. in "Decolorization of heat-treatment liquor of waste sludge by a bioreactor using polyurethane foam-immobilized white rot fungi equipped with an ultramembrane filtration unit" also propose immobilization of the ligninolytic fungi *Coriolus hirsutus* to improve decolorization of dye-containing effluents. In this work, authors claim that fungus immobilization on porous polyurethane solve the difficulty in the solid/liquid separation after effluent treatment. Susla and Svobodová (Susla and Svobodová, 2008) in "Effect of various synthetic dyes on the production of manganesedependent peroxidase isoenzymes by immobilized Irpex lacteus*"* reported the higher efficiency of dye decolourization of PUR foam immobilizated cultures of *I. lacteus* than free-cells cultures because of the higher production of MnP.

None of the above mentioned scientific papers or patents refer to PUR composition or structure as a factor to be considered for ligninolytic enzymes production and most do not even include this information. Instead they analyze the effect of different culture medium components, environmental factors or reactor design on ligninolytic enzymes production.

Ligninolytic enzymes are highly versatile in nature and they find application in a wide variety of fields of industry and environment: textile, food, paper and wood processing, bioremediation, pharmaceutical, chemical industries and nanobiotechnology. They have been proposed for bioconversion of lignocelluloses into value added-products (e.g. biofuels and chemical feedstocks in biorefinery), pulp delignification, dye bleaching, improvement of the whiteness in conventional bleaching of cotton, biostoning, detoxification of wastewaters and other bioremediation processes, preparation of musts and wines, polymerization of lignin and lignosulphonates, detergent manufacturing, modification of biopolymers for the production of derivatives suitable for incorporation as copolymers in synthetic polymers for paints, plastics, and films, tailoring of lignocellulosic materials by laccase-assisted biografting of phenols and other compounds and transformation of antibiotic and steroids (Rodriguez Couto and Toca Herrera, 2006; Maciel et al., 2010).

A few laccases are at present in market for textile, food and other industries, and more candidates are being actively developed for future commercialization. Related to textile bleaching, in 1996 Novozyme (Novo Nordisk, Denmark) launched DeniLite, a laccase-based product for denim finishing (Dickson et al., 1997). In 2001, the company Zytex (Zytex Pvt Ltd. Mumbai, India) also developed a laccase-based formulation capable of degrading indigo dye. The trade name of the product is Zylite. Laccases can generate a desired worn appearance on denim by bleaching indigo dye. A laccase has recently been commercialized (Suberzyme ®) for preparing cork stoppers for wine bottles (Conrad et al. 2000)

The biotechnological significance of these enzymes has led to a drastic increase in its demand in the recent time. Industrial enzyme market is valued at $2.000 million per annum with a potential annual growth rate of 3 to 5%. Laccase stake in this market is about 4% thus making it a potential $800 million market cap mainly in the textile industry and enzyme bleaching of pulp (Dewan, 2011).

Energy-saving of ligninolytic-based biocatalysts fit well with the development of highly efficient, sustainable, and eco-friendly industries. Also, there is a broad field of investigation that is almost entirely open to new findings and it is quite reasonable that many new applications will be found in the near future.

### Disclosure of the invention

The invention is characterized by use of polyurethane rigid and flexible foams (PUR), which are obtainable from wood origin raw materials - tall oil derived polyols or lignin, as immobilizing support for microorganisms that produce ligninolytic enzymes. The offered PUR (see claims from 1 to 8) and method of their production (see claims 11 to 13) ensure that produced open cell PUR structure as composite with natural fibres or produced by lignin facilitates colonization of microorganism inside the foam and stimulates the production of ligninolytic enzymes. There is verified that these properties of produced foams together with the presence therein of natural fibres or lignin source, which induce ligninolytic enzymes production by the microorganisms, makes these foams a valuable and completely new material for the application.

### Used Tall oil (TO).

For implementation of the embodiment of the invention Tall oil (TO) was used that was a mix of different fatty (C₁₂ ― C₂₂) and rosin acids (C₂₀). Common samples of used TO are shown below, where (a) is fatty acid ― oleic acid (C₁₈H₃₄O₂), and (b) is rosin acid ― abietic acid (C₂₀H₃₀O₂) (1):

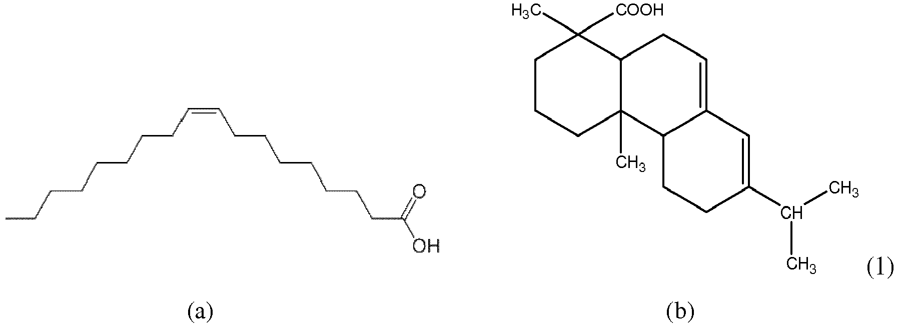

Firstly, optimum synthesis conditions of polyols suitable for obtaining polyurethane rigid foams (PUR) were determined. The synthesis process was controlled from the triethanolamine (acid value) or diethanolamine (acid value and NH value) conversion degree. It should be mentioned that the temperature in the esterification process is higher (170°C) than in the amidization process (145°C). The structure of polyols was characterized from FTIR spectroscopy data, which were obtained with a *Perkin-Elmer SpectrumOne* (UK) spectrometer at a resolution of 4 cm⁻¹, 32 scans.

The typical used formulations were polyols from TO, and a higher functional polyether polyol based on sorbitol Lupranol 3422 (it contains only secondary hydroxyl groups, OH Number 490 mg KOH/g) - from *BASF* (Ludwigshafen, Germany). As additives' surfactants, NIAX Silicone L620, the catalyst was NIAX Catalyst E-A-1 (all NIAX products from *Momentive Performance Materials* (Columbus, USA) and Polycat 5 (from *Air Products* (Utrecht, the Netherlands)). As an isocyanate, polymeric diphenylmethane diisocyanate - IsoPMDI 92140, supplied by *BASF* (Ludwigshafen, Germany) was used.

### Used natural fibres (NF)

NF utilized in the study were wood fibres from *LaSole* (Percoto, Italy) (WoodLs) and *Rettenmaier* (Rosenberg, Germany) (WoodRet). Characteristics of the used fibres are given in the Table 1. However other types' wood fibres can be used as well.

**Table 1**

| Fibres | Supplier | Water content, % | Wax, % | Lignin, % | OH content, mgKOH/g | Dimensions L, µm | Dimens -sions L, µm | Aspect ratio |
|---|---|---|---|---|---|---|---|---|
| WoodRet | *Rettenmaier* | 4.5 | 0.9 | 28.7 | 322 | 363 | 64 | 6.7 |
| WoodLS | *LaSole* | 4.9 | 3.5 | 29.6 | 244 | 286 | 80 | 5.5 |

### Used Lignin

Any lignin from sulphite pulping process, semi-mechanical pulping process, thermomecanical pulping process, semi-chemical pulping process, solvent process, steam explosion pulping process and biomass pulping process can be used, also any kind of chemically modified lignin and directly any kind of liquefied lignin obtained by different liquefaction process of wood or lignocellulosic feedstock can also be used. The present invention is also characterized in that lignin is dissolved in a polyol and is used in a form of polyol solution. The polyol used for lignin dissolution according to this invention may be, for example, a low molecular weight polyol such as ethylen glycol, diethylen glycol, triethylen glycol trimethylolpropane, glycerol; a polyether polyol such as polyethylene glycol with different molecular weight. Samples of the mixture of lignin, at predetermined weight ratio, placed in glass flasks were subjected to the microwave treatment in a microwave oven just after the addition of liquefying polyols. Parameters such as heat power (from 100 to 600 W) and the duration of heating between 1-10 min were varied. The liquefied lignin is rich in polyols, which can be directly used as feedstock for making polymers without further separation or purification. Isocyanate reactive chain extender agents used in the present invention may be, for example, a polyether polyol such as polypropylene glycol triol, polycaprolactone, and hydroxyl group containing liquid polybutadiene with suitable functionality and high molecular weight; a renewable resources based polyols like castor oil, soybean oil, cardanol, cashew nutshell oil. Suitable polyisocyanates to be used as component in accordance with the present invention, include for example, NCO prepolymers and liquid polyisocyanates. Diisocyanates preferred for the process according to the invention are those represented by the formula R(NCO)₂ in which R represents an aliphatic or aromatic hydrocarbon group. Example of the suitable organic diisocyanates include 1,4-tetramethylene diisocyanate, 1,6-hexamethylene diisocyanate, 2,2'-, 2,4'-and/or 4,4'-diphenylmethane diisocyanate, any type of mixture of these isocyanate and similar isocyanate are suitable for the present invention. It is preferred that the polyisocyanates of the present invention are aromatic polyisocianates. Some examples of suitable aromatic polyisocyanates are 2,4- and/or 2,6-toluene diisocyanate (TDI), 2,2'-, 2,4'-and/or 4,4'-diphenylmethane diisocyanate (MDI). Most preferred polyisocyanates comprise a blend of the 2,4- and 2,6-isomers of toluene diisocyanate; an isomeric mixture of diphenylmethane diisocyanate comprising the 2,2'-, 2,4'-and/or 4,4'-isomers; and a polymethylene poly(phenylisocyanate) comprising a monomer content of from 40% to 100% and a content of higher homologues of MDI of from 60% to 0%. Suitable blowing agents to be used as a component in accordance with the present invention include compounds such as, for example, water, carbon dioxide, low boiling hydrocarbons. Water and carbon dioxide are more preferred blowing agents, with water being the most preferred. In accordance with the present invention, the quantity of blowing agent used is typically that will produce foams having a density ranging between 60 kg/m³ and 200 kg/m³. Suitable surfactants to be used as component in accordance with the present invention include, for example, any of the known surfactants which are suitable for production of polyurethane foams. These include for example, but are not limited to silicone-type surfactants, fluorine-type surfactants, organic surfactants, etc. Some examples of suitable surfactant include those compounds commercially available from General Electric, Air Products, etc. such as sold as DABCO DC2525, DC5160, DC 5164, etc. In accordance with the invention, one or more catalysts are used. Any suitable urethane catalyst may be used, including the tertiary amine compounds and organometallic compounds. Examples of suitable tertiary amine catalyst include triethylendiamine, dimethylcyclohexylamine, tetra-methylenediamine, bis (dimethylaminoethyl)ether, pentamethyl diethylenetriamine and diethylethanolamine. Example of organometallic catalysts include organomercury, organolead and organotin catalysts, with organotin being preferred. Suitable organotin catalysts include preferably tin(II) salts of carboxylic acid, such as tin (II) acetate, tin (II) octoate, tin (II) laurate, as well as tin (IV) compounds, such as dibutyltin dialurate, dibutyltin maleate and dibutyltin dichloride. Typical amounts are about 0.01 to about 4 pphp (part per 100 parts by weight of polyol), and preferably about 0.03 to about 2.0 pphp of catalyst. The flexible foams of the present invention are prepared by mixing together one or more isocyanates with the isocyanate reactive component, blowing agents, catalysts and surfactants. After mixing, the foaming mixture may be deposited into an open container and be allowed to rise freely (free-rise process). The foaming mixture may also be deposited into a mould that is then closed thus forcing the foam to take on the shape of the mould (controlled-rise process). The hydroxyl number for the liquefied lignin was determined according to the following method: 1g of liquefied lignin was esterified for 20 min at 110°C with 25ml of a phthalation reagent, a mixture of 22.5 g phtalic anhydride, 3.63 g imidazol, and 150 g dioxane. After that the excess reagent was titrated with 1 M sodium hydroxide solution. The hydroxyl number was calculated from the difference in titration of blank and the sample solutions. To evaluate the density, a sample piece of 30mmx30mmx30mm was prepared. The apparent density a of three pieces for each sample was measured and the mean value was calculated. The compression force deflection values of the foams were measured with the procedure prescribed in ASTM D3574C. FTIR (Fourier Transformed Infrared), dynamic-mechanical analysis (DMTA) and scanning electron microscopy (SEM) were performed on samples produced.

In the present invention lignin is liquefied preferentially by microwave technology, and water is the single blowing agent for the production of flexible foams by lignin. The application of PUR as support for growth of microorganisms that produce lignolytic enzymes is highly innovative.

### Immobilization of the fungus on rigid and soft PUR

The microorganism used according to embodiment of this invention was *Phanerochaete flavido-alba* ATCC 60998, which is available from American Type Culture Collection. Any lignocellulolytic microorganism is also suitable for this application, especially fungi. Examples of fungi that can be used in the process of this invention include *Phanerochaete chrysosporium, Trametes versicolor, Phlebia radiata* and *Cerrena multicolor,* which produce ligninolytic enzymes (laccase and/or lignin peroxidase and/or manganese peroxidase). The present invention is also characterized by the use of immobilizing support made of soft or rigid PUR foams for fungal growth, which are resistant to microbial attack, but contain ingredients that stimulate ligninolytic enzyme production. This process comprises two successive steps, each of them being performed under different culture conditions. Ligninolytic enzymes are usually produced under stress conditions when the nutrient supply is reduced and the fungus begins to starve, also some compounds may stimulate or protect the enzyme. Thus, production typically proceeds through alternating cycles of growth and ligninolytic-enzymes production which are controlled by supplying the fungus with a growth-producing medium followed by an enzyme-producing medium which is deficient in certain nutrients or includes components that stimulate the enzyme production or protect it once produced.

In the present invention the first step of immobilization involves the culture of cells (e.g. *P. flavido-alba*) in a rich medium that promotes growth rather than production of enzymes. Examples of culture media for this step are potato dextrose broth (PDB), malt extract (ME) or yeast-malt-peptone-glucose (YMPG). The foams are semi-submerged in growth medium and act as a solid support for the growth of *P. flavido-alba.* This allows access of nutrients and a continuous and natural supply of oxygen.

A second step involving culture of the mycelium formed during the first step in a production medium to enhance the production of ligninolytic enzymes, during which the whole of the culture medium of the first step is replaced with a production medium. The production medium comprises carbon and nitrogen sources, inorganic salts, trace elements and it is supplemented with enzyme inducer/stabilizer. The carbon source used comprises glucose at a concentration of 10g/L, but other assimilable carbon sources can be used, alone or mixed, examples of such carbon sources include glycerol, maltose, starch, cellobiose, and cellulose. The nitrogen source can consist of aminoacids, nitrate, ammonium, peptone, yeast extract or a mixture of these nitrogen sources. The nitrogen sources used were a mixture of these nitrogen sources at concentration between 0,2-5g/l. The inorganic salts comprise potassium, calcium, magnesium salts and trace elements are mainly composed of iron sulphate, zinc sulphate, manganese sulphate, cobalt sulphate and copper sulphate. The culture medium is supplemented by adding (0.4-0.5g/l) at least one component which activates the production of enzymes or protect them, this component can consist of one of the constituents or a mixture of them. Preferably, this constituent comprises veratryl alcohol and Tween 80, which, in this invention are used at a concentration of 0.42g/l and 0.5g/L, respectively, or 2'-azinobis-(3-ethylbenzthiazoline-6-sulfonate (ABTS) at a concentration of 0.55g/L. Other suitable inducers are ferulic acid, guaiacol, syringic acid, vanillin, tannic acid or synthetic dyes such as poly R-478 or Remazol Brilliant Blue R (RBBR).

Growth of microorganism was measured in terms of the dry weight of the mycelium after it was dried overnight at 105°C. An average predetermined foam cube dry weight was subtracted from the weight of the foam plus mycelium.

Ligninolytic enzymes Lignin peroxidase (LiP) and/or Laccase (Lac) were measured in the extracellular culture at different time cultures in order to determine the peak production. Lignin peroxidase (LiP) was determined by the oxidation of veratryl alcohol to veratraldehyde. The assay reagent is composed of 2mM veratryl alcohol in the presence of 0.4mM hydrogen peroxide in25mM sodium tartrate buffer at pH 2.5. The reaction is monitored spectrophotometrically at 310nm. The molar extinction coefficient of veratraldehyde at this wavelength is 9300M⁻¹cm⁻¹. Laccase (Lac) was analyzed by monitoring the oxidation of syringaldazine 0.0216mM in citrate-phosphate buffer 100mM at pH 5.2. The reaction is monitored spectrophotometrically at 525nm. The molar extinction coefficient of oxidized syringaldazine at this wavelength is 65.000 M⁻¹cm-1. Enzymatic activity was expressed in U/L with 1 U defined as 1 µmol of the substrate oxidized per min. All chemicals were supplied by Sigma-Aldrich.

Method of immobilization of microorganisms in polyurethane foams containing natural fibre. Method for implementation of the invention is described step by step in five examples:
- synthesis of Tall oil amide with rosin acid content 2% (step 1, version 1) is described in Example 1;
- synthesis of Tall oil amide with rosin acid content 20% (step 1, version 2) is described in Example 2;
- synthesis of Tall oil ester with rosin acid content 2% (step 1, version 3) is described in Example 3;
- production of polyurethane rigid foams (step 2) is described in Example 4;
- immobilization on rigid foams of microorganisms, which produce ligninolytic enzymes (step 3) is described in Example 5.
- production of ligninolytic enzymes by immobilized microorganism on rigid foams (step 4) is described in Example 6.

### Example 1

Synthesis of Tall oil amide with rosin acid content 2% (step 1. version 1). Raw materials used for synthesis and their characteristics are shown in Table 2.

**Table 2**

| **Raw materials** | **Molar ratio** | **m, kg** |
|---|---|---|
| Tall oil with 2% rosin acid content | 1 M | 20 |
| Diethanolamine | 1,15 M | 8,39 |

For carrying out of synthesis Tall oil and diethanolamine was weighted and poured into reactor, afterwards heating was begun. Synthesis was done in ideal mixing type reactor at 140°C but because of temperature inertia in reactor temperature fluctuated from 138 to 144°C. In reactor was secured inert gas atmosphere. Gas flow was supplied for bottom of reactor to help mix substances and evaporate water. Beginning of synthesis was established when first amount of water condensed. Process was continued till products acid value had dropped to 5mgKOH/g; afterwards product was vacuumed for 1.5 hour at 200 mgHg, to ensure that all water was separated. Kinetics of synthesis is shown in Table 3.

**Table 3**

| **Synthesis time, h** | **Synthesis pressure, bar** | **Acid value, mgKOH/g** | **Separeted V_{H2O}, ml** |
|---|---|---|---|
| 5 | 1 | 11.3 | 1200 |
| 1, 5 | 0.2 ― 0.3 | 4.57 | 1380 |

Characteristics of the product obtained are shown in Table 4.

**Table 4**

| **Parameter** | **Value** |
|---|---|
| Acid value, mgKOH/g | 4,19 |
| (OH+NH) value, mgKOH/g | 298,4 |
| NH value, mgKOH/g | 45,64 |
| H₂O, mas.% | 0,17 |

### Example 2

Synthesis of Tall oil amide with rosin acid content 20% (step 1. Version 2). Raw materials used for synthesis and their characteristics are shown in Table 5.

**Table 5**

| **Raw materials** | **Molar ratio** | **m, kg** |
|---|---|---|
| Tall oil with 20% rosin acid content | 1 M | 20 |
| Diethanolamine | 1,15 M | 8,24 |

For carrying out of synthesis Tall oil and diethanolamine were weighted and poured into reactor, afterwards heating was begun. Synthesis was done in ideal mixing type reactor at 140°C; because of temperature inertia in reactor temperature fluctuated from 138 to 144°C. In reactor inert gas atmosphere was secured. Gas flow was supplied for bottom of reactor to help mix substances and evaporate water. Beginning of synthesis was established when first amount of water condensed. Process was continued till products acid value hah dropped to 5mgKOH/g; afterwards product was vacuumed for 1.5 hour at 200 mg Hg, to ensure that all water was separated. Kinetics of synthesis is shown in Table 6.

**Table 6**

| **Synthesis time, h** | **Synthesis pressure, bar** | **Acid value, mgKOH/g** | **Separeted V_{H2O}, ml** |
|---|---|---|---|
| 5 | 1 | 15,24 | 1200 |
| 2 | 0.2 ― 0.3 | 4,15 | 1320 |

### Characteristics of product obtained are shown in Table 7

**Table 7**

| **Parameter** | **Value** |
|---|---|
| Acid value, mgKOH/g | 4,15 |
| (OH+NH) value, mgKOH/g | 275,0 |
| NH value, mgKOH/g | 57,30 |
| H₂O, mas.% | 0,15 |

### Example 3

Synthesis of Tall oil ester with rosin acid content 2% (step 1. version 3). Raw materials used for synthesis and their characteristics are shown in Table 8.

**Table 8**

| **Raw materials** | **Molar ratio** | **m, kg** |
|---|---|---|
| Tall oil with 2% rosin acid cont. | 1 M | 17,0 |
| Triethanolamine | 1,33 M | 11,6 |

For carrying out of synthesis Tall oil and triethanolamine was weighted and poured into reactor, afterwards heating had begun. Synthesis was done in ideal mixing type reactor at 175°C but because of temperature inertia in reactor temperature fluctuated from 170 to 180°C. In reactor inert gas atmosphere was secured. Gas flow was supplied for bottom of reactor to help mix substances and evaporate water. Beginning of synthesis was established when first amount of water condensed. Process was continued till products acid value had dropped to 5mgKOH/g; afterwards product was vacuumed for 1.5 hour at 200 mg Hg, to ensure that all water was separated. Kinetics of synthesis is shown in Table 9.

**Table 9**

| **Synthesis time, h** | **Synthesis pressure, bar** | **Acid value, mgKOH/g** | **Separeted V_{H2O}, ml** |
|---|---|---|---|
| 3 | 1 | 5,76 | 940 |
| 2 | 0.2 ― 0.3 | 3,49 | ~1000 |

Characteristics of product obtained are shown in Table 10.

**Table 10**

| **Parameter** | **Value** |
|---|---|
| Acid value, mgKOH/g | 3,49 |
| (OH+NH) value, mgKOH/g | 337,3 |
| H₂O, mas.% | 0,15 |

### Example 4

Pproduction of polyurethane rigid foams (step 2). PUR samples were obtained by mechanically mixing appropriate amounts of IsoPMDI 92140 and the polyol system (polyols, surfactant, catalysts, blowing agent and natural fibres for 10-15 s. The unreacted mixture was poured into a plastic mould (20 x 30 x 10 cm) for free foaming. The polymerization reaction took place at room temperature for all obtained samples and was completed in about 3-5 min; the samples were conditioned before tests for 24 h. The content of different components in the composites was calculated on the basis of initial weights. The typical formulations used in this study were polyols from TO, and a higher functional polyether polyol based on sorbitol Lupranol 3422 (it contains only secondary hydroxyl groups, OH Number 490 mg KOH/g) - from *BASF* (Ludwigshafen, Germany). As additives' surfactants, NIAX Silicone L620, the catalyst was NIAX Catalyst E-A-1 (all NIAX products from *Momentive Performance Materials* (Columbus, USA)), Polycat 5 (from *Air Products* (Utrecht, the Netherlands)) and Potasium acetate (30%0 in diethylenglycol (PCCatTKA30) (Performance Chemical Handels GmbH, Hamburg, Germany). As an isocyanate, polymeric diphenylmethane diisocyanate - IsoPMDI 92140, supplied by *BASF* (Ludwigshafen, Germany) was used. Compositions for the preparation of foams for immobilization experiments are shown in Table 11.

**Table 11**

| Component | Open cell foams |
|---|---|
| | *Parts by weight* |
| Tall oil amide from Example 2 | 85 |
| Lupranol 3422 | 10 |
| Glycerol | 5 |
| Water | 3 |
| EA-1 | 0.2 |
| Polycat 5 | 0.2 |
| K Ac | 0.4 |
| L620 | 1.5 |
| NF | 0 ... 9 |

| | *Isocyanate index* |
|---|---|
| IsoPMDI 92140 | 160 ... 170 |

The obtained foams have density 40 ± 5 kg/m³, closed cell content 10 ± 2%, compressive strength 0.15 ― 0.25 MPa. The content of renewable raw material in ready foams varied from 24.9 to 27.7%.

Compositions for the preparation of foams according to the present invention may contain the following Polyol and Isocyanate components: tall oil amide ― form 70 to 85 parts by weight; higher functional polyether polyol based on sorbitol, containing only secondary hydroxyl groups ― from 5 to 10 parts by weight; glycerol ― from 2 to 5 parts by weight; water - from 2 to 3 parts by weight; tertiary amine as catalyst ― from 0.2 to 0.4 parts by weight; potassium acetate as catalyst ― 0.2-0.5 parts by weight; open cells forming surfactant ― from 1.0 to 1.5 parts by weight; natural fibres from 3 to 9 parts by weight; said components as Polyol component and Polymeric MDI having functionality 2.7, NCO group content 31.5% - from 160 to 170 parts by weight as Isocyanate component.

### Example 5.

Immobilization on rigid PUR foams of microorganisms that produce ligninolytic enzymes (step 3). Eight different rigid PUR with a density of 40±5 kg/m³ were used as a support for fungal cell immobilization. They included foams prepared as in example 4 with open cells without NF reinforcement or reinforced with 9% NF (as in Example 4) of either Woodrett or WoodLS (as in Table 1). Prior to use, the foams were rinsed with 70% (v/v) ethanol for 1 h and washed thoroughly three times with distilled water. The foams were then dried and 5 pieces (2 x 2 x 1 cm) were weighed and placed in a 250 mL Erlenmeyer flask containing 50 mL of growth medium potato dextrose broth (PDB). After sterilization by autoclaving at 121°C for 20 min, the flask was inoculated with two 1 cm² plugs of a seven-days-old culture of fungi on potato dextrose agar (PDA). A control without PUR was included as a free-cell culture reference. Incubation was done in triplicate at 30°C without shaking. Conidiospores attached easily to the PUR and growth took place throughout most of the support after 11 days.

### Example 6

Production of ligninolytic enzymes by immobilized microorganism on rigid PUR (step 4). For enzyme production, the immobilized cultures were exposed to a medium amended with an inducer for ligninolytic enzyme production (ABTS). The total liquid volume of immobilized cultures from Example 5 was aseptically decanted and the flasks were re-filled with 25 mL of fresh enzyme production medium (Table 12)

**Table 12**

| Component | Value per liter |
|---|---|
| Glucose | 10 g |
| Peptone | 5 g |
| Yeast Extract | 2 g |
| ABTS (2'-azinobis-(3-ethylbenzthiazoline-6-sulfonate) | 0.55 g |
| Mineral solution* | 70 mL |

| | |
|---|---|
| *Mineral solution in g/l: 1.5 g nitrilacetic acid at 99%; 3g MgSO₄.7H₂O; 0.5g MnSO₄.H₂O; 1g NaCl; 0.1g FeSO₄.7H₂O; 0.1g CoSO₄; 0.1g CaCl₂.2H₂O; 0.1g ZnSO₄.7H₂O; 0.01g CuSO₄.5H₂O; 0.01g AlK(SO₄)₂.12H₂O; 0.01g H₃BO₃; 0.01g Na₂MoO₄.2H₂O) | |

Cultures were incubated at 30°C without shaking for 13 days. Lignin-degrading enzymes (Lac and LiP) were analyzed in the extracellular culture. The production of Lac and LiP by *P. flavido-alba* on immobilized cultures with different materials and its comparison with the enzyme levels obtained in free-cells cultures (control level) were carried out (Table 13). In general, the enzyme production was enhanced when the fungus was immobilized on PUR. Foams with NF were superior for Lac production than foams without NF. By using foams with either WoodRet or WoodLS as reinforcement fibres, the Lac activity was increased 7-fold in comparison with the case of the control culture. The maximum Lac activity (140 U/L) was obtained in cultures immobilized on the foam reinforced with WoodRet. This result is in agreement with the possible induction of the enzyme activity by the lignocellulose present in these foams. In the case of LiP production, the effect of the foam structure was not so pronounced, but again the higher enzyme values were obtained in immobilized cultures on the foams reinforced with NF. The highest LiP activity (190 U/L) was obtained in the PUR reinforced with WoodLS, whose levels were more than 10-fold higher than those obtained in free-cell cultures.

**Table 13**

| Immobilization support | Enzyme activity | | Biomass |
|---|---|---|---|
| | Lac (U/L) | LiP (U/L) | (mg/piece) |
| PUR-without NF | 58.46 | 100.61 | 86 |
| PUR-9%WoodLS | 115.85 | 190.35 | 102 |
| PUR-9%WoodRet | 140.00 | 157.10 | 115 |
| Without immobilization support (free cells) | 23.34 | 48.26 | |

Method of immobilization of microorganisms in flexible polyurethane foams produced by lignin. The method for implementation of the invention is described step by step in five examples:
- synthesis of flexible polyurethane by lignin and PPGtriol (step 1) is described in Example 7
- synthesis of flexible polyurethane by hydroxypropylated lignin and PPGtriol (step 1) is described in Example 8;
- synthesis flexible polyurethane by hydroxypropylated lignin and castor oil (step 1) is described in Example 9;
- production of polyurethane flexible foams (step 2) is described in Example 10;
- immobilization on flexible foams of microorganisms (step 3) that produce ligninolytic enzymes is described in Example 11.
- production of ligninolytic enzymes by microorganism immobilized on flexible foams (step 4) is described in Example 12.

### Example 7

Synthesis of flexible polyurethane by lignin and PPGtriol (step 1). Lignin was Protobind™ 1000, provided by GreenValue SA, it is soda pulping lignin, derived from non-wood plant sources, such as flax, wheat straw and other grasses. The first step performed was the dissolution of lignin, previously dried for 24 hours in an oven with air circulation at a temperature of 80 °C. Samples of the lignin, at predetermined weight ratio, placed in glass flasks were subjected to microwave treatment in a microwave oven just after the addition of liquefying polyols: glycerol and polyethylene glycol 400 (PEG 400) y Sigma Chemicals. Parameters such as heat power (from 100 to 600 W) and the duration of heating, between 1-10 min, were varied. An additional compound capable of reducing the viscosity of the solution and increasing the flexibility of the structure, reducing the glass transition temperature of the final material, was added such as polypropilenglycol triol (PPG triol) from Sigma Chemicals, used as received. The solution obtained by mixing and dissolving the lignin and the chain extender can be used as a reagent, after determining the concentration of hydroxyl groups.

ISO 116/1, a polymeric diphenylmethane diisocyanate (MDI) has 25.7% part by weight of NCO content, it was provided by ELASTOGRAN. Isocyanate in excess of that needed to react with the OH groups on the polyols reacted with distilled water to form CO₂, which acted as the only foam blowing agent.

The catalysts used were by Air Products and belong to two different series: classic catalysts and new generation catalysts (NE), with low emission of amine. The classic catalysts consisted of non-reactive amines that can be released from the foams, once it is produced. They were DABCO 33LV (gelling) and DABCO BL11 (blowing), and their composition is shown in Table 14. The series of catalysts NE comprised: DABCO NE1070 (gelling) and DABCO NE300 (blowing), this series of catalysts can reduce emissions because the molecules that act as catalysts can chemically bind themselves to the polyurethane matrix. To ensure uniformity of structure and enhance the cells opening DABCO DC2525 was used, it is a silicone surfactant made from 70% by weight of polysiloxane. Catalysts composition is shown in Table 14.

**Table 14**

| **Type** | **Composition** |
|---|---|
| DABCO 33LV | triethylenediamine (67% by weight) dipropylene glycol (33% by weight) |
| DABCO BL11 | bis(dimethilamminoethil)ether (70% by weight) dipropylene glycol (30% by weight) |
| DABCO NE1070 | dimethylaminopropyl urea, 3- (30 - 70% by weight) |
| DABCO NE300 | N-[2-[2(dimethylamino)ethoxy]ethyl]-N-methyl-1,3-propanediamine (90% by weight) |

All the ingredients except the MDI were weighted in a polypropylene cup and mixed for 30 seconds. At the end of the mixing period, MDI was added and the mixing continued for another 10 seconds. The foams were allowed to cure for 24 hours before testing. Composition of soft PU samples produced by lignin and PPGtriol is reported in Table 15.

**Table 15**

| Code | Type of lignin | chain extender | lignin/chain extender | NCO/OH | Catalysts | %Lignin |
|---|---|---|---|---|---|---|
| P-PPG-C3 | Protobind 1000 | PPG triol | 1.4 | 0.80 | Classic | 10.00 |
| P-PPG-D3 | Protobind 1000 | PPG triol | 1.0 | 0.69 | Classic | 11.9 |
| P-PPG-E3 | Protobind 1000 | PPG triol | 0.8 | 0.63 | Classic | 13.35 |
| P-PPG-F3 | Protobind 1000 | PPG triol | 1.4 | 0.81 | NE | 10.00 |

### Example 8

Synthesis of flexible polyurethane by hydroxypropylated lignin and PPGTriol (step 1). Hydroxypropylation is a widely accepted method to improve the functionality of biomasses, The process was performed in aqueous alkali at room temperature at moderate pH (only the phenolic OH groups are ionized) and produced a well-soluble lignin derivative of lower glass transition temperature compared to the pure lignin. The same procedure as in example 1 was applied. Composition of soft PU samples produced by hydroxypropylatedlignin and PPGTriol is reported in Table 16.

**Table 16**

| Code | Type of lignin | chain extende | lignin/chain extender | NCO/OH | Catalysts | %Lignin |
|---|---|---|---|---|---|---|
| HP-PPG-A3 | Hydroxypropylated protobind 1000 | PPG triol | 1,45 | 0.82 | Classic | 6.70 |
| HP-PPG-B3 | Hydroxypropylated protobind 1000 | PPG triol | 1,45 | 0.77 | NE | 6.70 |
| HP-PPG-C3 | Hydroxypropylated protobind 1000 | PPG triol | 1.40 | 0.80 | Classic | 10.0 |
| HP-PPG-D3 | Hydroxypropylated protobind 1000 | PPG triol | 1.0 | 0.69 | Classic | 11.9 |
| HP-PPG-E | Hydroxypropylated protobind 1000 | PPG triol | 0.8 | 0.63 | Classic | 13.35 |
| HP-PPG-F | Hydroxypropylated protobind 1000 | PPG triol | 1.4 | 0.81 | NE | 10.00 |
| HP-PPG-G3 | Hydroxypropylated protobind 1000 | PPG triol | 1.0 | 0.70 | NE | 11.78 |

### Example 9

Synthesis of flexible polyurethane by hydroxypropylated lignin and Castor oil (step 1). The same procedure of example 2 was applied but for the Chain extender that was Castor oil purchased from Sigma Chemicals and used as received. All the ingredients except the MDI were weighted in a polypropylene cup and mixed for 30 seconds. At the end of the mixing period, MDI was added and the mixing continued for another 10 seconds. The foams were allowed to cure for 24 hours before testing.

Composition of soft PU samples produced by hydroxypropylatedlignin and Castor oil is reported in Table 17.

**Table 17**

| Code | Type of lignin.. | Chain extende | Lignin/chain extender | NCO/OH | Catalysts | %Lignin |
|---|---|---|---|---|---|---|
| HPCO-A-3 CR | Hydroxypropylated protobind 1000 | castor oil | 1,45 | 0.75 | Classic | 6 (40.3) |
| HPCO-B-3 CR | Hydroxypropylated protobind 1000 | castor oil | 1,45 | 0.72 | NE | 6.3 (42.7) |

Sample HPCO-A-3-CR and HPCO-B-3-CR have respectively a percentage of 40.3% and 42.7% of renewable material taking in consideration both lignin and castor oil.

### Example 10

Production of polyurethane flexible foams (step 2). The flexible foams are produced in plastic containers (85x85x55 mm). One of the most used techniques to obtain polyurethanes is the one-shot technique, which consists of a very efficient mixing, in one step only, in a short time, of all the raw materials involved in polyurethane fabrication: isocyanate, oligo-polyol, chain extenders or crosslink silicon emulsifiers, blowing agents, catalysts. In order to simplify the procedure with the use of many components, a master-batch, that is a mixture of the components that do not react with each other, (e.g., oligo-polyol, water, chain extender, catalysts, etc.), was made before foaming. Then it was possible to use only two components: one is the polyolic component (called component A or formulated polyol, containing a mixture of all raw materials except for the isocyanate, in the proportions needed) and the second component is the isocyanate (called component B or isocyanate component). The polyurethane that results is a consequence of the very efficient contact between the isocyanate component and the polyolic component.

### Example 11

Immobilization on flexible foams of microorganisms that produce ligninolytic enzymes (step 3). Forteen different soft PUR were used as a support for *P. flavido-alba* immobilization. They included foams produced as in examples 2 and 3 containing different lignin type, chain extender and catalyst (as in Tables 15, 16 and 17). The foams were used as it (not washed soft PUR) or rinsed with 70% (v/v) ethanol for 1 h and washed thoroughly three times with distilled water (washed soft PUR). Two different foam sizes were tested, big size 3 x 2.8 x 2.8, and small size 1.7 x 1.7 x 1.7. The foams were dried at 45°C and 1 piece was weighed and placed in a 500 mL NALGENE bottle containing 50 mL of growth medium potato dextrose broth. After sterilization by autoclaving at 121°C for 20 min, the growth medium was inoculated with one 1 cm² plug of a seven-days-old culture of fungi on potato dextrose agar. A control without soft PUR was included as a free-cell culture reference. Incubation was done at 30°C without shaking.

### Example 12

Production of ligninolytic enzymes by immobilized microorganism on soft PUR (step 4). For enzyme production, the immobilized cultures were exposed to a medium amended with inducers for ligninolytic enzyme production (Tween 80 and veratryl alcohol) (Table 18). The total liquid volume of immobilized cultures was aseptically decanted and the bottles were re-filled with 50 mL of fresh enzyme production medium (Table 18).

**Table 18**

| Component | Value *per Liter* |
|---|---|
| Glucose | 10 g |
| Amonium tartrate | 0.2 g |
| Sodium tartrate | 4.6 g |
| Amonium nitrate | 2.6 g |
| KH₂PO₄ | 2 g |
| MgSO₄.7H₂O | 0.5 g |
| CaCl₂.2H₂O | 0.1 g |
| Tween 80 | 0.5 g |
| Veratryl Alcohol | 0.42 g |
| Thiamine―HCl | 1 mg |
| Mineral solution* | 70mL |

| | |
|---|---|
| *Mineral solution in g/L: 1.5 g nitrilacetic acid at 99%; 3g MgSO₄.7H₂O; 0.5g MnSO₄.H₂O; 1g NaCl; 0.1g FeSO₄.7H₂O; 0.1g CoSO₄; 0.1g CaCl₂.2H₂O; 0.1g ZnSO₄.7H₂O; 0.01g CuSO₄.5H₂O; 0.01g AlK(SO₄)_{2.}12H₂O; 0.01g H₃BO₃; 0.01g Na₂MoO₄.2H₂O | |

Cultures were incubated at 30°C without shaking for 11 days. Laccase was analyzed in the extracellular culture. Flexible foams with small size (1.7 x 1.7 x 1.7) were superior for laccase production than big size foams (Table 19). This may be related with the higher specific surface available in the small size pieces in comparison with big size pieces. Small size flexible foams made by hydroxypropylated lignin and Castor oil (HP-CO) gave higher laccase production (average of 66.87 U/L) than those made of lignin and PPGtriol (P-PPG) hydroxypropylated lignin and PPGTriol (HP-PPG) (average of 44 U/L).

**Table 19**

| | Laccase (U/L) | |
|---|---|---|
| Samples | Big size foam (3 x 2.8 x 2.8 cm) | Small size foams (1.7 x 1.7 x 1.7 cm) |
| HP-CO | 8.26 | 66.87 |
| HP-PPG | 4.78 | 44.59 |
| P-PPG | 15.20 | 42.78 |

In general laccase production was higher in small size washed foams cubes than in non-washed ones (Table 20). Immobilization of fungi led to an increase in laccase production of more than 8-fold in some foams specimens in comparison with laccase values obtained in non-immobilized cultures. The best foams for ligninolytic microorganisms immobilization and subsequent laccase production were HPCO-A-3-CR, HP-PPG-A3 and P-PPG-E3, by using these supports laccase production reached values of 94, 82 and 83 U/L, respectively.

**Table 20**

| Immobilization support | Laccase (U/L) | |
|---|---|---|
| | Non-washed soft PUR | Washed soft PUR |
| HPCO-A-3-CR | 36.05 | 94.17 |
| HPCO-B-3-CR | 69.55 | 67.73 |
| HP-PPG-A3 | 62.53 | 81.49 |
| HP-PPG-B3 | 17.06 | 53.04 |
| HP-PPG-C3 | 57.95 | 59.14 |
| HP-PPG-D3 | 61.38 | 42.06 |
| HP-PPG-E3 | 47.81 | 54.32 |
| HP-PPG-F3 | 13.46 | 14.96 |
| HP-PPG-G3 | 2.60 | 26.50 |
| P-PPG-C3 | 47.98 | 51.33 |
| P-PPG-D3 | 30.39 | 54.48 |
| P-PPG-E3 | 37.17 | 82.67 |
| P-PPG-F3 | 42.89 | 55.82 |
| P-PPG-G3 | 17.18 | 35.74 |
| Without immobilization support (free cells) | 12.49 | |

### Relevant prior art:

Borges da Silva E.A., Zabkova M., Araújo J.D., Cateto C.A., Barreiro M.F., Belgacem M.N., Rodrigues A.E., An integrated process to produce vanillin and lignin-based polyurethanes from Kraft lignin, Chemical Engineering Research and Design, 2009, 87, pp. 1276―1292.
Dewan, S.S., Enzymes in Industrial Applications: Global Markets. BCC Research, 2011, BIO030F
Dosoretz, C.G., Rothschild, N., Hadar, Y. 1993. Overproduction of lignin peroxidase by Phanerochaete chrysosporium (BKM-F-1767) under nonlimiting nutrient conditions. Applied and Environmental Microbiology, 1993, 59, pp. 1919-1926.
Duran, N., Rosa, M.A., Dannibale, A., Gianfreda, L. 2002. Applications of laccases and tyrosinases (phenoloxidases) immobilized on different supports: A review. Enzyme and Microbial Technology, 2002, 31, pp. 907―931.
Fujita M., Era, A., Ike, M., Soda, S., Miyata, N., Hirao, T. (2000). Decolorization of heat-treatment liquor of waste sludge by a bioreactor using polyurethane foam-immobilized white rot fungi equipped with an ultramembrane filtration unit. Journal of Bioscience and Bioengineering, 2000, 90, pp. 387-394.
Guo A., Javni, I., Petrovic Z., Rigid Polyurethane Foams Based on Soybean Oil, Journal of Applied Polymer Science, 2000, 77, pp. 467―473.
Guo A., Lava S., Zlatanic A., Petrovic Z., Structure-property relationships in different types of vegetable oils, Abstracts of Papers, 224th ACS National Meeting, Boston, MA, United States, August 18-22, 2002, BTEC-006, American Chemical Society, Washington.
Hatakeyama H., Hatakeyama T., Environmentally compatible hybrid type polyurethane foams containing saccharide and lignin components, Macromolecular Symposia, 2005, 224, pp. 219-226.
Hill K., Fats and oils as oleochemical raw materials, Pure Appl.Chem., 2000, 72, pp.1255-1264
Hinz W., Kraus R., Larbig H., Guettes B., Scherzer D., Tischer G., Homogeneous stable polyhydroxyl compound containing lignin with good processing properties, 1997, Patent application number: DE 19548350.
Jenkines R.C., Polyurethane carpet backings made using hydroxymethylated polyester polyols, 2006, Patent number: US 7794814
Kurple K.R., Lignin based polyols, 1997, Patent number: US 6025452.
Kwon, H.-S., Chung, E., Oh, J., Lee, Ch.-H., and Ahn, I.-S. 2008. Optimized Production of Lignolytic Manganese Peroxidase in Immobilized Cultures of Phanerochaete chrysosporium. Biotechnol. Bioproc. Engin. 13: 108-114;
Maciel, M. J. M., Silva, A. C. E., & Ribeiro, H. C. T. (2010). Industrial and biotechnological applications of ligninolytic enzymes of the basidiomycota: a review. Electronic Journal of Biotechnology, 13, 6.
Mahmoud, D.A.R. and Helmy, W.A. 2009. Potential application of immobilization technology in enzyme and biomass production J. Appl. Sci. Res., 5: 2466-2476;
Petrovic Z., Polyurethanes from Vegetable Oils, Polymer Reviews, 2008, 48, pp. 109-155.
Rodriguez Couto, S., and Sanromán, M.A. 2005. Application of solid-state fermentation to ligninolytic enzyme production. Biochem. Engin. J. 22: 211―219;
Rodriguez Couto, S., and Toca Herrera, J.L. 2006. Industrial and biotechnological applications of laccases: A review. Biotechnology Advances 24, 500― 513;
Susla, M., and Svobodová, K. 2008. Effect of various synthetic dyes on the production of manganesedependent peroxidase isoenzymes by immobilized Irpex lacteusWorld J. Microbiol. 24: 225-230;
Susla, M., Novotný, C., and Svobodová, K. 2007. The implication of Dichomitus squalens laccase isoenzymes in dye decolorization by immobilized fungal culturesBioresour. Technol., 98: 2109-2115.

### Synthesis of Polyurethane from Renewable Resources

Blount D.H., Flame retardant urea bio-based urethane composition, 2006, Patent number: US 7129291.
Glasser W.G., De Olivera W., Kelley S.S., Nieb L.S., Method of producing prepolymers from hydroxyalkyl lignin derivatives, 1992, Patent number: US 5,102,992.
Glasser W.G., De Olivera W., Kelley S.S., Nieb L.S., Method of producing star-like polymers from lignin, 1991, Patent number: US 5,066,790.
Glasser W.G., Hsien-Hsiang Hsu O., Polyurethane intermediates and products and methods of producing same from lignin, 1977, Patent number: US 4017474.
Hatakeyama H., Hirose, S., Lignin-based polyurethane and process for producing the same, 2005, Patent application number: US 20050014919.
Hirose S., Yano S., Hatakeyama H., Nakamura K., New polyurethane and processing for preparing same, 1989, Patent number: EP 342781.
Jarre W., Wrumb R., Hydrophobic polyurethane foams, their manufacture and use, 1980,
Patent Number US4237237. Kelly P.T., Frisch K.C., Sendijarevic V., Biodegradable polyurethane foams prepared from renewable resources, 2001, Patent Application number: EP 1162222 A2.
Klesczewski B., Triouleyre S., Gossner M., Meyer-Ahrens S., Mosbach-Rosenberger A., Visco-elastic polyurethane foam with castor oil, 2009, Patent application number: US 20090264547.
Kurth T.M., Improved cellular plastic material, 2000, Patent number: WO 0015684.
Mueller H.-P., Sirinyan K., Kapps M., Rigid hydrophobic polyurethanes, 2000, Patent number US 6,075,064
Nascimento J.F., Pachekoski W.M., De Lello Vicino J.R., Composition for preparing a biodegradable polyurethane-based foam and a biodegradable polyurethane foam, 2009, Patent application number: US 20090253816.
Petrovic Z., Javni I., Zlatanic A., Guo A, Modified vegetable oil-based polyols, 2010, patent number US 8,153,746 B2.
Reese J.R., Moore M.N., Wardius D.S., Hager S.L., Novel polyether polyols based on cashew nutshell liquid, a process for the production of these polyether polyols, flexible foams produced from these polyether polyols, and a process for the production of these foams, 2008, Patent application number: US 20080139685.
Reese H.-J., Heimpel F., Forster H., Pressurized, blowing agent-containing isocyanate semiprepolymer mixtures based on lignin-polyether polyols and their use for producing polyurethane foams, 1998, Patent number: US 5,834,529.
Scherbel K., Ebert K., Appenroth H., von Malotki P., Preparation of polyurethane rigid foams, 1997, Patent number: US 5,688,835.

### Immobilization of microorganisms and enzyme applications

Asther, M., Capdevila, C., Corrieu, G. 1992. Microbial method for producing lignin peroxidase. US patent 5,153,121.
Belinky, P., Lasser, H., Dosoretz, C.. 2009. Methods for producing lignin peroxidase and its use in skin and hair lightening. US patent application 2009/0041692 A1
Conrad, L.S., Sponholz, W.R., Berker, O.: Treatment of cork with a phenol oxidizing enzyme. US 6,152,966 (2000).
Dickson, G.A., Gray, D.R. 1997. Process and composition for decorating a dyed cloth fabric. US Patent 5,593,458
Moukha, S., Sigoillot, J.-C., Frasse, P., Asther, M. 1999. *Phanerochaete chrysosporium* strains CNCM I-1511, I-1512 and I-1513 for producing lignin peroxidase and manganese peroxidase. US patent 5,972,672.

## Claims

1. Polyurethane rigid or flexible foam obtained from wood origin raw materials as composite for use as a support for immobilization of microorganisms, which produce ligninolytic enzymes.

2. Polyurethane rigid foam according to claim 1, wherein said foam is obtained from Tall oil derived polyols, where said polyurethane rigid foam is containing natural fibres.

3. Foam according to claim 2, wherein Tall oil is a mix of fatty acids and rosin acids, where fatty acid is preferably oleic acid (C₁₈H₃₄O₂) and rosin acid is preferably abietic acid (C₂₀H₃₀O₂).

4. Foam according to claim 2 or 3, wherein natural fibres are selected from wood wood or cellulose fibres having dimensions from 64 to 363 µm, aspect ratio from 5.5 to 6.7, water content from 4.5 to 4.9%, lignin content from 28.7 to 29.6%, OH content from 244 to 322 mgKOH/g.

5. Polyurethane flexible foam according to claim 1, wherein said foam is obtained from lignin derived polyols.

6. Foam according to claim 5, obtained by polycondensation of a polyol solution containing liquefied lignin with a polyisocyanate, where lignin is being extracted from wood residues, wood flour, wood chips, saw dust, paper mill residues, or any other known lignin containing wood origin raw material.

7. Foam according to claim 6, wherein the liquefied lignin is obtained by microwave heating of lignin and glycerol or polyethylene glycol.

8. Foam according to claim 6 or 7, wherein the polyisocyanate is aromatic polyisocyanate.

9. Use of foams according to any claim from 1 to 8 as a support for immobilization of microorganisms, which produce ligninolytic enzymes.

10. Use of the microorganism selected from the group consisting of *Phanerochaete flavido-alba* ATCC 60998, *Phanerochaete chrysosporium, Trametes versicolor, Phlebia radiata* and *Cerrena multicolor* on foams according to any claim from 1 to 8 for immobilization of microorganisms and producing ligninolytic enzymes.

11. Method for production of polyurethane rigid foam as composite according to any claim from 1 to 4 for immobilization of microorganisms and production of ligninolytic enzymes therein, the method comprising the steps of:
- synthesis of Tall oil amide from tall oil with rosin acid content from 2 to 20%;
- production of polyurethane rigid foam by mixing the following components: tall oil amide ― form 70 to 85 parts by weight; higher functional polyether polyol based on sorbitol, containing only secondary hydroxyl groups ― from 5 to 10 parts by weight; glycerol ― from 2 to 5 parts by weight; water - from 2 to 3 parts by weight; tertiary amine as catalyst ― from 0.2 to 0.4 parts by weight, potassium acetate as catalyst ― 0.2-0.5 parts by weight; open cells forming surfactant ― from 1.0 to 1.5 parts by weight; natural fibres from 3 to 9 parts by weight and Polymeric MDI having functionality 2.7 and NCO group content 31.5%) ― from 160 to 170 parts by weight;
- immobilization on rigid foams obtained microorganisms, which produce ligninolytic enzymes.

12. Method for production of polyurethane flexible foam as composite according to claim 1 or any claims from 5 to 8 for immobilization of microorganisms and production of ligninolytic enzymes therein, the method comprising the steps of mixing together isocyanate reactive component and one or more isocyanates, wherein:
- isocyanate is diisocyanate represented by formula R(NCO)₂, in which R represents an aliphatic or aromatic hydrocarbon group,
- isocyanate reactive component is obtained by mixing together lignin containing polyol, chain extender, blowing agent, catalyst and surfactant, wherein lignin containing polyol is obtained by dissolving lignin in a polyol; blowing agent is selected from the group consisting of water, carbon dioxide, low boiling hydrocarbons; catalyst is urethane catalyst, e.g. tertiary amine compounds and organometallic compounds.

13. The method according to claim 12, wherein dissolving of lignin in a polyol is being made by microwave treatment in a microwave oven.
